Europäisches Patentamt

European Patent Office

Office européen des brevets

(19)

(11) Publication number: **0 040 964**
**A1**

# EUROPEAN PATENT APPLICATION

(21) Application number: **81302273.8**

(22) Date of filing: **21.05.81**

(51) Int. Cl.³: **G 01 N 33/70, C 12 N 9/12**

(30) Priority: **23.05.80 US 152915**
**10.04.81 US 252775**

(43) Date of publication of application: **02.12.81**
**Bulletin 81/48**

(84) Designated Contracting States: **BE CH DE FR GB IT LI NL SE**

(71) Applicant: **Burnam, Michael Howard, 4061 Hayvenhurst Drive, Encino California (US)**
Applicant: **Shell, William Elson, 956 Chantilly Road, Bel Air California (US)**

(72) Inventor: **Burnam, Michael Howard, 4061 Hayvenhurst Drive, Encino California (US)**
Inventor: **Shell, William Elson, 956 Chantilly Road, Bel Air California (US)**

(74) Representative: **Perry, Robert Edward et al, GILL JENNINGS & EVERY 53-64 Chancery Lane, London WC2A 1HN (GB)**

(54) **Protein enzyme derivative.**

(57) The B subunit of creatine kinase has been made substantially free of albumin and substantially enzymatically inactive. Antibodies recognising the subunit can be raised by inoculating a host with a suitable immunogen and collecting serum or plasma from the host. The subunit may be labelled, e.g. with a radioisotope. The labelled subunit and antibodies may be packed separately in a diagnostic kit.

EP 0 040 964 A1

PROTEIN ENZYME DERIVATIVE

This invention relates to a protein enzyme derivative, i.e. a purified enzyme subunit, which may be labelled, to antibodies recognising it, to methods of assaying it, and to diagnostic kits comprising the labelled subunit and a respective antibody.

Creatine kinase, hereinafter referred to as CK, is a protein enzyme found in muscle and other tissues. There are at least three presently accepted isoenzyme forms of CK. Each isoenzyme molecule is comprised of two subunits; these two subunits are called M and B, respectively. Thus, there can be a CK isoenzyme with two M subunits (hereinafter called MM), an isoenzyme with two B units hereinafter called BB), and an isoenzyme with one B and one M subunit (hereinafter called MB). The MM isoenzyme is characteristic of skeletal muscle, the BB isoenzyme is predominantly localized to brain but is also found in various smooth muscle including the gut and the uterus. The MB isoenzyme is thought to be specific for the human heart muscle (myocardium).

Blood tests for total CK enzyme activity have been used in diagnosis and estimation of severity of myocardial infarcts since 1962, Hughes, Clin. Chem. Acta., 7:597:1962. It has been known since the early 1970's that the MB isoenzyme is released into the circulation following a myocardial infarct. See, e.g., Klein, et.al., Cardiovascular Research 7, 412-418 (1973) or Roe, et.al., J. Lab. Clin. Med., 80:577:1972. However, since it is presently accepted that most of the CK in the plasma or serum of normal man is MM (approximately 98%), a specific change in the MB fraction could not be accurately measured because of the coincident measurement of the MM background.

Subsequent electrophoretic separation of total serum CK into its isoenzymes has been used as a diagnostic test for myocardial infarcts. However, the procedure is non-quantitative and only provides an estimate of the relative percentage of the isoenzyme. Moreover, electrophoretic systems are relatively insensitive at the lower range of MB activity thereby resulting in frequent false negative tests. Also, at high MB activities the relative percentage of MB is frequently overestimated leading to numerous false positive tests.

More recently, several other methods have been developed in search of better specificity and sensitivity for MB activity as well as ease of operation. One such class of methods involves separation on ion exchange resins formed in a column or attached to glass beads. Although this method theoretically achieves greater analytic sensitivity, in practice this sensitivity has not been achieved because the isoenzymes are incompletely removed from the resin. Moreover, increased specificity has not been achieved because the MM fraction frequently tails into the MB fraction, especially at high MM concentrations. Since in patients with disease MM represents about 85% to 99% of total activity, even a 1-2% carryover of MM activity in the MB zone can give false positive tests. Accordingly, the current CK isoenzyme fractionation techniques have not been found to be completely acceptable.

Immunoassay procedures have also been under study and reported upon in the literature. An immunoassay procedure used in measuring MB activity values has recently been marketed. This procedure utilizes an antibody allegedly specific for MM, thereby removing MM activity from the serum sample. The CK activity measurable is therefore attributed to MB since the BB isoenzyme had been thought to be restricted to brain tissue, thereby not interfering with the measurement of MB, Merck, GMBH, U.S. Patent 4,067,775. Other procedures involving immunological techniques have focused upon the theoretical cross-reactivity of an antibody made to BB with the B-subunit of MB, Roberts, et.al., Science, 194:855:1976. The rationale accompanying this approach is two-pronged: (1) A rise in BB concentration will not be observed in serum of a patient having a suspected myocardial infarct, (2) the BB antibody will have sufficient cross-reactivity to bind the B-subunit of MB, thereby providing a means for evaluating MB release into blood following a myocardial infarct. Currently, this approach is being used in research areas utilizing radioimmunoassay as a potentially promising tumor marker since prostatic carcinoma and adenocarcinoma give off substantial quantities of BB which are recognized by an antibody to "B"-CK, Clinical Chemistry News, 5, p. 1, Sept. 1979. Additionally, a "B-CK" radioimmunoassay kit is on the marketplace for myocardial infarcts and prostatic carcinoma detection. However, these assays have significant drawbacks, including, inter alia, the lack of sufficiently pure standards, relatively unstable radiolabeled ligand, and lengthy incubation periods.

Additionally, and perhaps of even greater significance, these assays only measure enzymatically active B-subunit. The presence of an immunoassay, particularly a radioimmunoassay, which solves these problems would be a significant step forward in this area.

BRIEF SUMMARY OF THE INVENTION

In accordance with this invention, an antibody recognizing B-subunit of CK and having a high affinity constant has been raised. A highly purified B-subunit of CK, essentially, if not totally, free of albumin and essentially enzymatically inactive has been prepared. This B-subunit of CK which is referred to hereinafter as CK-Bi when radiolabeled is of markedly increased stability.

A process of radiolabeling CK-Bi is novel. Another aspect of the invention is the preparation of the purified CK-Bi. An immunoassay utilizing the above-identified CK-Bi antibody, labeled CK-Bi and purified CK-Bi as a standard has evolved. The immunoassay is readily marketed as a mercantile unit, preferably a kit, as well as a laboratory service.

Set forth below in more detail are various aspects of this invention.

An aspect of this invention is an immunogen designated CK-Bi which is essentially free of albumin and essentially inactive enzymatically.

Another aspect of this invention is an antibody which recognizes CK-Bi, said antibody being prepared by (1) inoculating a host animal with an immunogen comprising heterologou. CK-Bi and (2) collecting the serum or plasma from said host animal. Such an antibody has been shown to have high affinity for its antigen as demonstrated by a very short incubation period during immunoassay.

Another aspect of the invention is the steps necessary to prepare the essentially albumin free and essentially enzymatically inactive B-subunit of CK, i.e., CK-Bi.

A still further aspect of the invention is a method for radiolabeling CK-Bi so as to produce a stable, labeled product.

A still further aspect of the invention is a method for the assay of CK-Bi in a sample which comprises incubating said sample with a known amount of labeled CK-Bi and an antibody which recognizes CK-Bi, separating the free CK-Bi and free labeled CK-Bi from the bound CK-Bi and bound labeled CK-Bi, measuring the amount of labeled CK-Bi in either the free or antibody-bound materials and determining the concentration of CK-Bi in the sample by reference to a standard curve.

A still further aspect is a kit wherein the component parts are assembled into an immunoassay for CK-Bi concentration in a sample which comprises

(a) a container having therein a first reagent; and

(b) a separate second container having therein a second reagent;

(c) said first reagent comprising an antibody which recognizes CK-Bi; and

(d) said second reagent comprising labeled CK-Bi, said first reagent being intended to be contacted with the sample containing CK-Bi to be measured and with the second reagent in order to bind part of labeled CK-Bi or unlabeled CK-Bi to said antibody, the measurement value of the label being a function of the concentration of CK-Bi in said sample.

## DETAILED DESCRIPTION OF THE INVENTION

The sample of CK-Bi as being determined can be any biological fluid which contains detectable levels of CK-Bi.  Such fluids include blood, blood serum, blood plasma, tissue extracts and urine.

The immunogen which can be used in raising the antibody is prepared in a manner substantially different from known art methods. Rather than seeking to preserve the enzymatic activity as done in the Roberts and Nuclear Medical Systems assays, procedures are taken which purposefully render the CK-BB isoenzyme, removed from source, enzymatically inactive or essentially inactive.  The source of the CK-Bi is any tissue or physiological fluid which has significant levels of the isoenzyme that is classically known as BB-CK.  Examples of such tissue and fluids are blood, amniotic fluid, seminal fluid, uterine tissues, prostatic tissues, cerebrospinal fluid, lung tissue, and breast tissue.  A preferred source of CK-BB is brain, more preferably human brain.  The brain is initially prepared so as to minimize possible contaminants of the CK-Bi, particularly blood components such as albumin.  Standard processing steps of preparing BB-Ck from brain are utilized with the additional following steps.

Freshly removed brain should have the blood thoroughly washed away and then have the external pia-arachnoid membranes stripped. These steps remove a significant amount of potentially contaminating albumin from the BB-CK preparation.  When brain tissue is utilized as the source of CK-Bi, it is recommended that the cerebral arteries be

0040964

perfused with phosphate buffered saline at 4° C for one hour in order to avoid contamination of the tissue with blood. At this point the brain may now be frozen prior to carrying out the modified Carlson procedure, Carlson, et.al., J. Molec. Cell. Cardiology 8, 159 (1976). Additional purification is performed by multiple reprecipitation with ethanol. This resultant preparation is essentially free of albumin as shown by electrophoresis and immunological reaction. Prior to the multiple reprecipitation with ethanol the BB-CK shows CK activity. After the multiple reprecipitation, the CK activity is essentially absent and the resultant protein is CK-Bi. This protein may be further purified using a linear salt gradient.

As noted herein CK-Bi may be isolated from various tissues including blood. We have found that CK-Bi isolated from blood and CK-Bi isolated from other tissues where there has been substantial contamination with blood components is glycosylated with an acid glycoside. When tissues other than blood are used as the source of CK-Bi, and one is careful to avoid blood contamination of the tissue, the CK-Bi is not glycosylated. The non-glycosylated and glycosylated CK-Bi have the same immunogenicity and either form of CK-Bi may be utilized in raising antisera, for radiolabeling, and as a standard although it is preferred that the glycosylated form be used in standardization.

The CK-Bi obtained following the multiple ethanol reprecipitation step as generally described above has both a 38,000 dalton and a 42,000 dalton component as measured on sodium dodecyl sulfate (SDS) polyacrylamide gel electrophoresis. The 38,000 dalton component is preferable for radiolabeling, as well as for immunization and standardization. Additional characteristics of non-glycosylated CK-Bi are an isoelectric point of about 5.9 to 6.2 as measured by two dimensional isoelectric focusing on polyacrylamide gel and the tendency to aggregate in nondilute solutions. Glycosylated CK-Bi has a molecular weight of about 67,000 to 80,000 daltons as determined on Sephadex G-200 and about 42,000 on SDS polyacrylamide gel. Also glycosylated CK-Bi has an isoelectric point of 4.9 and is not precipitated by perchloric acid (PCA).

As used herein CK-Bi is taken tomean both glycosylated CK-Bi and non-glycosylated CK-Bi each of which is essentially free of albumen and essentially enzymatically inactive having less than one IU/mg of

0040964

enzyme activity.

It should be noted that the labeling of this preparation as CK-Bi is somewhat unique. Prior to this time, the identifying means of isoenzymes of creatine kinase has been enzymatic activity. This enzymatic activity is essentially absent in the preparation herein called CK-Bi. However, the preparation was derived from purified BB-CK, molecular weight determination of the CK-Bi is consistent with the accepted value of the monomeric unit of BB-CK, and the radioimmunoassay utilizing the labeled CK-Bi appears to successfully follow those clinical conditions commonly associated with the rise in levels of CK-Bi such as myocardial infarcts and prostatic carcinoma.

The antibody to the immunogen CK-Bi is prepared by injecting the immunogen into a host animal, preferably accompanied by an adjuvant, such as Freund's complete adjuvant, and an agent which enhances the inflammation response, such as mineral oil. Improved titres can be obtained by repeated injections over a period of time infrequently accompanied by an agent which nonspecifically enhances the immunologic response, such as pertussis vaccine. Suitable host animals for this purpose include mammals such as rabbits, horses, goats, guinea pigs, rats, cows, sheep and the like. The resulting antisera will contain antibodies which will recognize and bind CK-Bi with a binding specificity of 70 to 90%. We have also found that immunization with essentially pure CK-BB isoenzyme will result in antisera containing antibodies which will recognize and bind CK-Bi, such antisera having a binding specificity for CK-Bi of about 12 to 14%.

The labeled CK-Bi is used in competitive binding with CK-Bi contained in the fluid or tissue sample in the immunoassay. The label is a substance which is detectable by a variety of physical methods using standard instrumentation. Labels such as radioisotope, chromophore, fluorophore, red blood cell, electron spin resonance group, enzyme, etc., can be employed. Radioisotopes such as tritium ($^3$H), carbon 14 ($^{14}$C), or iodine isotopes such as $^{125}$iodine ($^{125}$I), iodine 131 ($^{131}$I) can be used and are preferred. $^{125}$I is the preferred radioisotope.

The radioisotopically labeled CK-Bi is prepared in a novel manner which elicits a stable radiolaeled material. The usual manner of attempting to radiolabel BB-CK, and then always an enzymatically active BB-CK, is through nonoxidative techniques such as the Bolton-Hunter ester method. These techniques have provided a nonstable radiolabeled material having a nonradiolabel-related shelf life of from about three to about seven days. Although potentially useful in the laboratory where it is actually prepared, such an unstable material would not be an effective commercial agent. Stable radiolabeled CK-Bi is prepared through oxidative techniques starting with CK-Bi. The basic Hunter and Greenwood method, Nature _194_, 495 (1962) and various modifications, that is chloramine T (N-chloro-4-methylbenzene-sulfonamide sodium salt) oxidation can be used. The preferred oxidative method utilizes 1,3,4,6-tetrachloro-3a,6a-diphenyl glycouril (TCDG). Labeled material of high specific activity having a shelf life of eight weeks or longer is obtained in this manner.

CK-Bi for use as mass standard is prepared by taking the protein from either the ethanol reprecipitation step or the linear salt gradient and further purifying it on a 65 cm long Sephadex G-200 or Sephacryl 300 column or by high performance liquid chromatography (HPLC). The protein standard which results is a single band on 6, 8 or 10% polyacrylamide gel, a single spot on two dimensional isoelectric focusing, a discrete peak on either Sephadex G-200 or HPLC. In addition, the standard is a single precipitant arc on immuno-electrophoresis against anti-CK-Bi antibody. Of course CK-Bi further purified in this manner may be used as the immunogen to raise antibody in the manner described hereinabove and as well is suitable for labeling, e.g., radiolabeling as described herein.

The immunoassay is preferably carried out as a radioimmunoassay. The radioimmunoassay is conducted as most immunoassays. The biological fluid or sample is incubated together with the radioisotopically labeled CK-Bi, preferably $^{125}$I labeled, and antibody recognizing CK-Bi in a buffer comprised of usual components useful in radioimmunoassay, for a significant period of time to achieve appropriate binding of antigen and antibody. After incubation, the labeled and unlabeled CK-Bi is separated from the free labeled and unlabeled CK-Bi by standard methods such as treatment with polyethylene glycol or contacting with a second antibody, the second antibody being an antibody specific for the gamma globulin of the host species in which the first antibody is raised. Such second antibody is prepared by standard techniques. Either the radioactivity from the bound or free material is measured and the concentration of CK-Bi present in the biological fluid sample is determined through reference to a standard curve. The standard

0040964

curve is derived by incubating fixed amounts of $^{125}I$ CK-Bi in the presence of a known concentration of antisera, with various known concentrations of CK-Bi and plotting the counts observed against such concentration.

As noted above non-glycosylated CK-Bi tends to aggregate under certain conditions and form dimers, trimers and tetramers of CK-Bi. The immunoassay functions to measure the monomeric CK-Bi.

The present invention is further illustrated by the examples which follow.

Example 1

Preparation of B-subunit of CK

Human brain tissue (100 gm) is obtained and stored frozen at -20° C until use. A modification of the method described by Carlson, et.al., is used to purify the CK-BB isoenzyme. The brain tissue is washed of blood with an isotonic solution and the pia-arachnoid membrane stripped. One hundred grams of tissue are processed at one time by homogenization in a precooled Waring blender containing 200 ml of ice cold 0.05 M Tris-HCl, pH 7.4, with 0.001 M 2-mercaptoethanol for three bursts of 15 seconds each. The resulting homogenate is centrifuged at 20,000 g in a Sorvall refrigerated centrifuge (GSA 9304) for 20 minutes; the supernate is retained. Cold ethanol (4° C) is added dropwise to the supernate to a final concentration of 60%, followed by stirring for 30 minutes at 2,100 x g in the same centrifuge and the pellet discarded. Cold 95% ethanol is again added dropwise to a final concentration of 70%, and the solution stirred at 4° C for an additional 30 minutes, followed by centrifugation at 2,100 x g for 15 minutes. The pellet from these procedures is resuspended in ice cold 0.05 M Tris-HCl, pH 7.4, with 0.001 M 2-mercaptoethanol in a Dounce homogenizer (10.5 ml resuspension buffer per 100 ml supernate). This material is centrifuged at 20,000 x g for 30 minutes and the pellet discarded. Protein concentration and CK activity are determined on the supernate.

DEAE Sephadex A-50 is swollen overnight in 0.05 M Tris-HCl, pH 7.4, containing 0.05 M NaCl and 0.001 M 2-mercaptoethanol. To the supernate from the previous ethanol precipitation, 4 M NaCl is added to yield a final concentration of 50 mM. This solution is then mixed with DEAE Sephadex A-50 in a proportion equal to 4.8 mg protein per milliliter of gel. The combined solution is then mixed by gentle agitation in a glass flask on ice for 30 minutes. The gel-supernate suspension is placed in a 1 x 10 cm column and allowed to settle. The void volume of buffer is discarded. The column is eluted with ice cold 0.05 M Tris-HCl, pH 7.4, containing 0.1 M NaCl and 0.001 M 2-mercaptoethanol. One milliliter aliquots are scanned for protein at 280 nM, and the elution terminated when no further protein is detected in the eluates. The elution buffer was then changed to ice cold 0.05 M Tris-HCl containing 0.3 M NaCl and 0.001 M 2-mercaptoethanol. The eluates are similarly scanned for protein at 280 nM. The peak fractions of protein are then assayed for activity and the 5 ml of highest CK activity retained. This preparation has an average specific activity of 200 IU/mg, and shows CK-BB enzyme activity on cellulose acetate electrophoresis.

Further purification is then performed by multiple reprecipitation with ethanol. 95% ethanol is added to the pooled eluates from the above procedure to a final concentration of 70%. The mixture is then stirred for 30 minutes at 0-4° C and then centrifuged for 20 minutes at 20,000 x g. The resultant pellet is washed with 10 ml of 95% ethanol (4° C) and recentrifuged at 32,000 x g for 15 minutes. This procedure is repeated a total of three times. The final pellet is assayed for CK activity and protein and then stored in a vacuum dessicator at -20° C. This preparation has a specific activity of less than one IU/mg. This material again demonstrates mobility of CK-BB on cellulose acetate electrophoresis. The CK-Bi thus obtained can be further purified, if desired, using a linear salt gradient, by procedures generally known in the art, tracking the position of CK-Bi in the gradient using a radioimmunoassay for CK-Bi or merely collect the 150 nM NaCl eluate. The thus obtained CK-Bi may be radiolabeled or may be used as the immunogen as described in the following examples with or without additional purification.

The essentially inactive B-subunit of CK prepared above, i.e., CK-Bi, is characterized by the following data. CK-Bi is electrophoresed on polyacrylamide gel in an Ames chamber by the method of Davis, Ann, N.Y. Acad. Sci. 121, 404 (1964). The final purified protein demonstrates a single protein stained band on a polyacrylamide gel in the presence of 0.1% sodium dodecyl sulfate (SDS) according to the method of Weber and Osborne, J. Biol. Chem. 244, 4406 (1969) modified by substituting heating of the protein in SDS dialysis buffer for

0040964

five minutes at 90° C rather than two hours at 37° C. This protein stained band is equivalent to a molecular weight of 38,000 to 42,000 daltons.

Example 1A

Purification of CK-Bi Protein from Human Serum Using Perchloric Acid (PCA) for Precipitant

PCA precipitant: Obtain human blood by venal puncture and allow it to clot for 30 minutes at 37° C. Centrifuge at 1000 x g (4° C) for 20 minutes. Harvest the serum in a Pasteur pipette. All subsequent steps should be performed on an ice cold bath or using a cooled centrifuge.

Dilute serum five times with distilled water. Allow the mixture to stir for 15 minutes. Add to the diluted serum 60% PCA to a final concentration of 3% PCA while slowly stirring the serum. A white precipitate should immediately form. Continue stirring for an additional 30 minutes, pour the solution into a high speed centrifuge tube and centrifuge at 8000 x g for 20 minutes. Collect the supernatant.

To the supernatant add two mM potassium hydroxide solution until the pH is adjusted to between 6 and 7. Stir the mixture for 20 minutes allowing a white precipitate to form. Stop stirring and allow the precipitate to settle without centrifugation. Decant the solution leaving behind the precipitate. Pour the solution into a dialysis bag and dialyze against 100 mM sodium chloride, 50 mM tris buffer, pH 7.4. Change the buffer three times. The preferred dialysis bag is the Spectrapor membrane with a 12,000 to 14,000 molecular weight cut-off.

DEAE elution: Make a 100 mililiter DEAE-Sephadex column in 100 mM sodium chloride, 50 mM tris buffer, pH 7.4. A column with a 2.5 cm diameter gives a sufficiently fast flow rate. Add the buffer exchange solution to the column. Elute with a linear salt gradient from 100 mM sodium chloride to 400 mM sodium chloride in a volume of at least 200 ml. Collect 1 ml samples and assay for CK-Bi using the CK-Bi radioimmunoassay. The protein is then concentrated to the desired concentration. This procedure results in a 40-50% yield using the CK-Bi standard as the mass standard.

This protein is an acid glycoprotein as defined by its reaction with a Schiff PAS stain on two-dimensional isoelectric focusing. In addition, this protein is not precipitated by 3% PCA, which is further evidence that it is a glycoprotein. Furthermore this protein has a significant affinity for concanavalin A.

## Example 2

### Radioiodination

Purified CK-Bi is radioiodinated according to a modification of the method of Hunter and Greenwood. Twenty-five µg of CK-Bi is dissolved in 25 µ of Tris 0.05 M, pH 8.0. Ten µl of this solution is then combined with 2 mCi $Na^{125}I$ (New England Nuclear, IMS 60, pH 7.4) followed by 25 µg of chloramine-T dissolved in 25 µl of phosphate buffer 0.4 M, pH 7.4. The reaction is allowed to proceed for 60 seconds at 4° C, interrupted by two brief vortex mixing steps. After 60 seconds, 50 µg of sodium metabisulfite dissolved in 0.05 M phosphate buffered saline, pH 7.4, is added to the reaction vessel along with 10 µl of 0.01 M KI. Separation of organified and nonorganified iodine is performed on a Biogel P-60 column (400 mesh) 1 x 10 cm, eluted with 0.05 M Tris, pH 8.0, containing 0.01 M EDTA at room temperature. One-half milliliter fractions are collected in 10% glycerine. The radioiodinated protein is aliquoted into glass tubes containing a small amount of anion exchange resin (Dowex-I) and stored at -20° C for further characterization and routine use.

A preferred method of radioiodinating CK-Bi is through the use of 1,3,4,6-tetrachloro-3a,6a-diphenyl glycouril. Conical borosilicate glass tubes are prepared containing 150 micrograms of the oxidant by its solubilization in chloroform, decanting into the tubes, and drying under nitrogen.

The iodination was performed at room temperature for 15 minutes. The reagents used are protein (10 micrograms in 0.05 M Tris, pH 8.0-20 microliters), 2 milicuries of iodine (pH 8 preferred), and 80 microliters of 0.4 M phosphate buffer, pH 7.4. The reactants are stirred by finger flicking every five minutes. After 15 minutes the reactants are placed on a biogel column (as stated above) followed by a rinse of the reaction vessel with 200 microliters of column eluant.

As determined by trichloroacetic acid (50 g IL) precipitation, 70% of the $^{125}I$ is incorporated into protein. In the presence of excess antiserum a maximum of 80-90% binding of $^{125}I$-CK-Bi can be demonstrated. Nonspecific binding of $^{125}I$-CK-Bi in the absence of antiserum averaged 1.5%. There was not a significant decrease in the percent binding of $^{125}$-CK-Bi after eight weeks other than that expected from the basis of $^{125}I$ decay.

When freshly prepared $^{125}I$-CK-Bi is studied by gel filtration on a Sephacryl S-300 column, a single peak at 38,000 to 40,000 daltons is shown, indicating that the molecular weight of the radioiodinated material is that of the basic subunit. When using a somewhat more concentrated preparation of $^{125}I$-CK-Bi a single peak at about 67,000 to 80,000 daltons is observed on Sephadex G-200. Additionally, when the same material is studied on a polyacrylamide gel with 0.1% SDS, a single protein fraction equivalent to 38,000 to 40,000 daltons is demonstrated.

When purified CK-Bi is placed on the Sephacryl S-300 column, four distinct molecular weight components are observed corresponding to approximately 40, 80, 120 and 160 x $10^3$ dalton indicating that purified BB-CK from brain exists in vitro as multiple aggregates of a basic 40,000 dalton (approximate) subunit. The predominant immunoreactive form appears to be in the 40,000 molecular weight componnt.

Example 3

Immunizations

Four adult new Zealand white rabbits are immunized. The primary immunization consists of 400 µg of CK-Bi dissolved in 0.5 ml of 0.05 M Tris, pH 8.0. The protein is allowed to dissolve in the buffer for 60 minutes at room temperature with constant stirring. This is combined with 0.5 of Freund's complete adjuvant and injected both intramuscularly and in multiple sites subcutaneously on the animal's back. Booster injections are performed consisting of 50 µg of protein in identical volumes of Tris buffer and adjuvant are performed at two month intervals. Incomplete Freund's adjuvant is used after an immunological response is detected. The animals are bled 10 to 14 days after each booster injection. All antisera are stored in aliquots at -20° C after adding 0.2 g of sodium azide per liter.

As indicated hereinabove, antisera which binds CK-Bi can also be raised, e.g., generally by the foregoing procedure when the rabbits are injected with the CK-BB isoenzyme.

Also, other inoculation regimens may be employed, e.g., the rabbits may be administered booster injections every three weeks for a total of four injections, and one may administer, intramuscularly, 0.1 cc pertussis vaccine along with the primary immunization.

Example 4

0040964

Radioimmunoassay Procedure

The assay procedure is performed as a double antibody technique with a 12-minute first antibody incubation and an 8-minute second antibody separation.

A. First antibody incubation: 0.225 ml total volume.
1. $^{125}I$-CK-Bi: 0.1 ml containing 50,000 cpm diluted in 0.5 M phosphate buffered saline (PBS), pH 7.4, containing 1% normal rabbit serum.
2. Sample: 0.025 ml; plasma samples are diluted 1/250 in PBS, pH 7.4.
3. Antibody: 0.1 ml of antibody sufficient to give 50-60% binding in 12 minutes; dilutions of antibody are made with PBS, pH 7.4.
4. For the Blank Tube, 1% normal rabbit serum is substituted for antiserum.
5. For the 100% Tube (Bo), 0.025 ml PBS is substituted for sample.
6. The order of addition is $^{125}I$-CK-Bi sample and antibody.

All additions were performed on ice and incubation time begins when placed in a water bath at 37° C.

B. Second antibody incubation: 0.925 ml total volume.
1. 0.1 ml goat anti-rabbit IgG obtained from Arnel Products Company diluted 1:6 in PBS buffer and 100 μl used per reaction tube; incubate five minutes at room temperature.
2. 0.6 ml 4.5% polyethylene glycol-6,000 in PBS; incubate additional three minutes at room temperature.

These additions are performed at room temperature and the tubes vortexed after both additions. After incubation the tubes are centrifuged for 10 minutes at 2,000 x g, the supernates decanted, and the radioactivity present in the residual pellets determined.

C. Antibody-bound radioactive counts are determined by counting centrifugation derived precipitates in a Searle model 1197 autogamma counter. All standards and unknowns are assayed in triplicate. The standards are prepared by diluting frozen aliquots of CK-Bi.

It should be noted that when taking human samples, particularly blood samples, the samples should be drawn into a tube containing substances such as ethylene glycol bis-)aminoethyl ether)-N,N' tetraacetic acid (EGTA) which functions as an anticoagulant and reducing agent. Blood drawn by venipuncture can be placed in EGTA which will provide a final concentration of 10 mM EGTA. This concentration of the reducing agent provides both anticoagulation and maximizes disaggregation of CK-Bi aggregate. It is preferred to run the immunoassay on a sample which contains the maximum amount of CK-Bi monomer. Although either blood serum or plasma may be used, it is preferred to use blood plasma in the immunoassay.

Example 5

Kit

The significant reagents for the immunoassay are assembled into a mercantile unit, specifically a kit, for marketing to qualified individuals to run as an assay. The kit should have as the minimum two separate containers, one container holding the antibody recognizing CK-Bi and the second container having CK-Bi generally as the monomer. Other separate containers may be there such as a container for CK-Bi to be used as a standard. Another container can contain the buffer in which the first incubation is carried out. Following the procedure of Example 4, a technician can correctly assay an unknown sample.

The immunoassay procedure described above can be used to assay for the presence of clinical conditions associated with a rise in CK-Bi values. The radioimmunoassay utilizing $^{125}I$ as a label has been used to identify acute myocardial infarction, prostatic carcinoma, and stroke, lung carcinoma, lymphoma, breast carcinoma, colonic carcinoma, and ovarian carcinoma. Its cross reactivity with respect to increased MM-CK measurements, associated with clinical events such as intramuscular injection, exercise, and noncardiovascular surgery, is essentially negligible. The assay has potential for identifying persons who are high risks for myocardial infarct and/or sudden death who suffer chest pain but are essentially normal in the other usual test systems such as enzyme activity, electrophoresis, and electrocardiogram.

0040964

CLAIMS

1.        The B subunit of creatine kinase, characterised in that it is substantially free of albumin and substantially enzymatically inactive.

2.        The subunit claimed in claim 1, characterised in that it shows two components, respectively having molecular weights of between 36,000 and 40,000 daltons and between 40,000 and 44,000 daltons, when subjected to electrophoresis on 7.5% polyacrylamide gel with 0.1% sodium dodecylsulfate.

3.        The subunit claimed in claim 1 or claim 2, characterised in that it shows a component having a molecular weight of from 67,000 to 80,000 daltons on Sephadex G-200.

4.        The subunit claimed in any preceding claim in aggregate form.

5.        The subunit claimed in any of claims 1 to 3 in monomeric form.

6.        The subunit claimed in any preceding claim which is glycosylated.

7.        The subunit claimed in any of claims 1 to 5 which is non-glycosylated.

8.        An antibody recognising the subunit claimed in any preceding claim, which has been prepared by (1) inoculating a host with an immunogen comprising the subunit claimed in any preceding claim; and (2) collecting the serum or plasma from the host.

9.        Serum or plasma which has been treated with the subunit claimed in any of claims 1 to 7.

10.       The subunit claimed in any of claims 1 to 7, characterised in that it is stable and labelled, e.g. with a radioisotope.

11.       The subunit claimed in claim 10, characterised in that the radioisotope is of iodine, e.g. $^{125}$I.

12.       A process for preparing the labelled subunit claimed in claim 10 or claim 11, which comprises labelling the subunit claimed in any of claims 1 to 7 under oxidising conditions, e.g. in the presence of chloramine T or

0040964

1,3,4,6-tetrachloro-3a,6a-diphenylglycouril.

13.     A method for assaying the subunit claimed in any of claims 1 to 7 in a sample, which comprises incubating the sample, a known amount of the labelled subunit, e.g. as claimed in claim 10 or claim 11, and an antibody which recognises the subunit, e.g. as claimed in claim 8 or claim 9; separating the free and bound subunit components; measuring the amount of the labelled subunit in either the free or antibody-bound components; and determining the concentration of the subunit in the sample by reference to a standard.

14.     A diagnostic kit, for use in an immunoassay for the subunit claimed in any of claims 1 to 7 in a sample, which comprises two separate containers, the first container containing an antibody recognising the subunit, e.g. as claimed in claim 8 or claim 9, and the second container containing the labelled subunit, e.g. as claimed in claim 10 or claim 11.

# EUROPEAN SEARCH REPORT

European Patent Office

0040964
EP 81 30 2273
Application number

## DOCUMENTS CONSIDERED TO BE RELEVANT

| Category | Citation of document with indication, where appropriate, of relevant passages | Relevant to claim |
|---|---|---|
| PX | CHEMICAL ABSTRACTS, vol. 94, no. 11, 16th March 1981, page 272, no. 79061t<br>Columbus, Ohio, U.S.A.<br>M.H. BURNAM et al.: "Oxidant-based radioiodination of "inactive" creatine kinase B protein"<br>& CLIN. CHEM. (WINSTON-SALEM, N.C.) 1981, 27(2), 351-353<br>* Abstract *<br>-- | 1,7-14 |
| PX | ABSTRACTS CIRCULATION, vol. 62, Supp III, October 1980, page III-215, no. 822<br>M.H. BURNAM et al.: "Development of a radioimmunoassay for inactive creatine kinase B monomer (CK-Bi)"<br>* Abstract *<br>-- | 1-3,5, 8,10, 11,13 |
| | ANNALS CLIN. LAB. SCI., 9/5, 1979, C.761, page 441<br>W.E. SHELL et al.: "The development and application of radioimmuno-assay specific for MB creatine kinase"<br>* Abstract *<br>-- | 10,11, 13 |
| A | ABSTRACTS CIRCULATION, vols. 57,58, supp II, October 1978, II-157, no. 611<br>M.H. BURNAM et al.: "Development of a radioimmunoassay specific for MB-creatine kinase"<br>* Whole abstract *<br>-- ./. | 8,13 |

## CLASSIFICATION OF THE APPLICATION (Int. Cl.³)

G 01 N 33/70
C 12 N 9/12

### TECHNICAL FIELDS SEARCHED (Int. Cl.³)

G 01 N 33/54
        33/56
C 12 N 9/12
C 12 D 13/10
C 12 Q 1/50
G 01 N 33/70

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 15-09-1981 | DE LUCA |

EPO Form 1503.1 06.78

0040964

European Patent
Office

EUROPEAN SEARCH REPORT

EP 81 30 2273
Application number
-2-

| | DOCUMENTS CONSIDERED TO BE RELEVANT | | CLASSIFICATION OF THE APPLICATION (Int. Cl.3) |
|---|---|---|---|
| Category | Citation of document with indication, where appropriate, of relevant passages | Relevant to claim | |
| PX | US - A - 4 267 271 (ROBERTS)<br><br>* Abstract; column 1, lines 10-14; column 3, lines 4-66; column 4, lines 48-56; examples 1-6; claims 1-3 * | 1,7-14 | |
| | -- | | |
| P | US - A - 4 237 219 (ROBERTS)<br><br>* Abstract; column 1, lines 10-14; column 3, lines 34-63; examples 1-6; claims 1-3 * | 7-14 | |
| | -- | | |
| D | US - A - 4 067 775 (WURZBURG)<br><br>* Abstract; column 2, lines 40-68; claim 1 * | 13 | TECHNICAL FIELDS SEARCHED (Int. Cl.3) |
| | -- | | |
| A | CLINICA CHIMICA ACTA, 58(1975), pages 223-232<br>E. JOCKERS WRETOU et al.: "Quantitation of creatine kinase isoenzymes in human tissues and sera by an immunological method"<br><br>* Pages 223-225 * | 8,13 | |
| | -- | | |
| P | CLINICAL CHEMISTRY, vol. 27, no. 1, January 1981, pages 83-87<br>Easton, Pennsylvania, U.S.A.<br>P. URDAL et al.: "Sensitive and specific radioimmunoassay for creatine kinase BB isoenzyme in serum, with use of an autoantibody"<br><br>* Whole article * | 8,10, 11,13 | |
| | ---- | | |